(19) Europäisches Patentamt
European Patent Office
Office européen des brevets

(11) Veröffentlichungsnummer: **0 515 942 A1**

# (12) EUROPÄISCHE PATENTANMELDUNG

(21) Anmeldenummer: **92108333.3**

(22) Anmeldetag: **18.05.92**

(51) Int. Cl.[5]: **A01N 43/54**

(30) Priorität: **29.05.91 DE 4117560**

(43) Veröffentlichungstag der Anmeldung:
**02.12.92 Patentblatt 92/49**

(84) Benannte Vertragsstaaten:
**DE DK ES FR GB GR IT NL SE**

(71) Anmelder: **BAYER AG**

**W-5090 Leverkusen 1 Bayerwerk(DE)**

(72) Erfinder: **Heinemann, Ulrich, Dr.**
**Am Sonnenhang 1**
**W-5653 Leichlingen 2(DE)**
Erfinder: **Paulus, Wilfried, Dr.**
**Deswatinesstrasse 90**
**W-4150 Krefeld 1(DE)**
Erfinder: **Ludwig, Georg-Wilhelm, Dr.**
**Kemmerhofstrasse 181**
**W-4150 Krefeld 1(DE)**

## (54) Verwendung von Pyridinylpyrimidinderivaten zum Schutz technischer Materialien.

(57) 2-(2-Pyridinyl)-pyrimidine besitzen eine ausgezeichnete biozide Wirkung und eignen sich daher hervorragend zum Schutz technischer Materialien.

EP 0 515 942 A1

Die Erfindung betrifft die Verwendung von 2-(2-Pyridinyl)-pyrimidinen als Mikrobizide zum Schutz technischer Materialien.

Aus der US-PS 3 296 272 ist die Verwendung von Alkylsulfinyl- und Alkylsulfonylpolyhalo-pyridinen als Mikrobizide zum Schutz technischer Materialien bekannt. Die antimikrobielle Wirksamkeit dieser Pyridinderivate ist jedoch auf bestimmten Indikationsgebieten nicht befriedigend; insbesondere macht sich ihre Hydrolyseempfindlichkeit bei der Anwendung in wäßrigen Systemen nachteilig bemerkbar, z.B. bei der Anwendung als Anstrichfungizide in Dispersionsfarben, insbesondere wenn diese alkalisch reagieren (siehe z.B. die Angaben in DE-OS 2 048 354, S. 14).

2-Mercapto-pyridin-N-oxid verfügt zwar über ein breites Spektrum antimikrobieller Wirkung und findet daher als Konservierungsstoff für wäßrige, funktionelle Flüssigkeiten Verwendung (K.H. Wallhäusser, (1988) "Sterilisation, Desinfektion, Konservierung", Georg Thieme Verlag, Stuttgart). Da es aber in Gegenwart von Sauerstoff und Licht sehr instabil ist und bei pH-Werten > 9,5 in die entsprechende weniger wirksame Sulfinsäure umgewandelt wird, eignet sich dieses Pyridinderivat nur in sehr begrenztem Umfang als Mikrobizid für den Schutz technischer Materialien. Verwendungsbeschränkend wirkt sich für 2-Mercapto-pyridin-N-oxid auch seine allgemeine Empfindlichkeit gegenüber Oxidations- und Reduktionsmitteln sowie die oft störende Bildung farbiger Chelate mit Metallionen, z.B. Eisen-oder Kupferionen, aus.

Aufgabe der Erfindung war daher das Bereitstellen von Mikrobiziden, die diese Nachteile nicht aufweisen, für den Schutz technischer Materialien.

Überraschenderweise wurde nun gefunden, daß bestimmte 2-(2-Pyridinyl)-pyrimidine ein sehr breites Wirkungsspektrum gegen solche Mikroorganismen besitzen, vor denen technische Materialien geschützt werden sollen (Bakterien, Pilze, Hefen, Algen). Darüber hinaus erweisen sich diese Verbindungen als sehr hydrolysestabil.

Gegenstand der Erfindung ist also die Verwendung von Verbindungen der Formel (I)

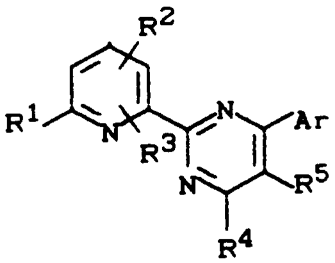

(I)

in welcher

| | |
|---|---|
| $R^1$ | für Wasserstoff, Halogen, Alkyl, Alkoxy, Alkylthio, Halogenalkyl, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist; |

in welcher weiterhin

| | |
|---|---|
| $R^1$ | für jeweils gegebenenfalls substituiertes Aryloxy, Arylthio, Aralkyloxy oder Aralkylthio steht, |
| $R^2$ und $R^3$ | unabhängig voneinander, gleich oder verschieden jeweils für Wasserstoff, Halogen, Alkyl, Halogenalkyl, Alkoxy, Alkylthio oder Alkoxycarbonyl stehen<br>oder |
| $R^2$ und $R^3$ | gemeinsam für eine Alkylenkette mit 3 bis 6 Kohlenstoffatomen stehen, die über die Ringpositionen 3 und 4 oder 4 und 5 verbunden sind, |
| $R^4$ | für Wasserstoff, Halogen, Alkyl, Halogenalkyl, Hydroxy, Alkoxy, Mercapto, Alkylthio, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist; oder |
| $R^4$ | für Alkenyloxy oder Alkinyloxy steht, |
| $R^5$ | für Wasserstoff oder Alkyl steht und |
| Ar | für unsubstituiertes oder substituiertes Aryl oder unsubstituiertes oder substituiertes Hetaryl steht, |

als Mikrobizide zum Schutz technischer Materialien.

Bevorzugte Substituenten bzw. Bereiche der in den oben und nachstehend erwähnten Formeln aufgeführten Reste werden im folgenden erläutert:

Für den Begriff Alkyl in den Definitionen von $R^1$, $R^2$, $R^3$, $R^4$ und $R^5$ in den allgemeinen Formeln steht geradkettiges oder verzweigtes Alkyl mit vorzugsweise 1 bis 6, besonders bevorzugt 1 bis 4 und insbesondere 1 oder 2 Kohlenstoffatomen. Beispielhaft seien Methyl, Ethyl, n-Propyl, i-Propyl, n-Butyl, sec-Butyl, i-Butyl, t-Butyl, n-Pentyl, i-Pentyl und t-Pentyl genannt.

Für den Begriff Alkenyloxy in der Definition von $R^4$ in den allgemeinen Formeln steht geradkettiges oder verzweigtes Alkenyloxy mit vorzugsweise 2 bis 6, besonders bevorzugt 2 bis 4 und ganz besonders bevorzugt 2 oder 3 Kohlenstoffatomen. Beispielhaft seien Vinyloxy, Allyloxy, 2-Propenyloxy, 1-Butenyloxy, 2-Butenyloxy, 3-Butenyloxy und 1-Methallyloxy genannt.

Für den Begriff Alkinyloxy in der Definition von $R^4$ in den allgemeinen Formeln steht geradkettiges oder verzweigtes Alkinyloxy mit vorzugsweise 2 bis 6, besonders bevorzugt 2 bis 4 und ganz besonders bevorzugt 2 oder 3 Kohlenstoffatomen. Beispielhaft seien genannt 2-Propinyloxy, 1-Butinyloxy, 2-Butinyloxy und 3-Butinyloxy.

Der Begriff (Di)alkylamino in den Definitionen von $R^1$ und $R^4$ in den allgemeinen Formeln steht für eine Aminogruppe mit 1 oder 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n-und i-Propyl genannt seien. Beispielhaft seien Methylamino, Dimethylamino, Ethylamino, Diethylamino, n-Propylamino, Di-n-propylamino, i-Propylamino und Di-i-propylamino aufgeführt.

Wenn im Falle von Dialkylamino die beiden Reste gemeinsam mit dem Stickstoffatom, an welches sie gebunden sind, einen Ring bilden, enthält dieser 5 bis 7, vorzugsweise 5 oder 6 Ringglieder. Der Ring kann 1 bis 3 Doppelbindungen enthalten, vorzugsweise ist er jedoch gesättigt. Der Ring kann ein weiteres Heteroatom, vorzugsweise Sauerstoff, Schwefel oder Stickstoff enthalten und durch 1 oder 2 Alkylgruppen vorzugsweise durch eine Alkylgruppe substituiert sein. Beispielhaft seien genannt: Pyrrolidino, Piperidino, Morpholino, N-Methylpiperazino, Thiomorpholino und 2,6-Dimethylmorpholino.

Unter dem Begriff unsubstituiertes oder substituiertes Aryl in der Definition von Ar in den allgemeinen Formeln ist Aryl mit vorzugsweise 6 bis 10 Kohlenstoffatomen im Arylteil zu verstehen. Beispielhaft und vorzugsweise seien unsubstituiertes oder substituiertes Phenyl oder Naphthyl, insbesondere Phenyl genannt.

Unter dem Begriff unsubstituiertes oder substituiertes Hetaryl sind vorzugsweise heteroparaffinische, heteroaromatische und heteroolefinische 5- bis 7-gliedrige Ringe mit vorzugsweise 1 bis 5, insbesondere 1 bis 4 Heteroatomen wie Sauerstoff, Schwefel oder Stickstoff zu verstehen, an welche jeweils gegebenenfalls ein weiterer gesättigter Carbocyclus mit vorzugsweise 5 oder 6 Kohlenstoffatomen, insbesondere mit 6 Kohlenstoffatomen anelliert ist. Beispielhaft und vorzugsweise seien genannt:

Pyrrolidinyl, Piperidinyl, Furyl, Thienyl, Pyrazolyl, Imidazolyl, 1,2,3- und 1,2,4-Triazolyl, Oxazolyl, Isoxazolyl, Thiazolyl, Isothiazolyl, 1,2,3-, 1,3,4-, 1,2,4- und 1,2,5-Oxadiazolyl, Azepinyl, Pyrrolyl, Isopyrrolyl, Pyridyl, Piperazinyl, Pyridazinyl, Pyrimidinyl, Pyrazinyl, 1,3,5-, 1,2,4- 1,2,3-Triazinyl, 1,2,4-, 1,3,2-, 1,3,6- und 1,2,6-Oxazinyl, Oxepinyl, Thiepinyl, 1,2,4-Diazepinyl, Chinolyl, Isochinolyl, Chinazolyl, Chinoxalyl, Tetrazolyl, Indolyl, Indazolyl, Benzimidazolyl, Benzothienyl, Benzofuryl, Benzthiazolyl, Benzoxazolyl, Thiadiazolyl, Isothiadiazolyl, Oxadiazolyl und Isoxadiazolyl.

Für den Begriff Halogenalkyl steht in den Definitionen von $R^1$, $R^2$, $R^3$ und $R^4$ in den allgemeinen Formeln vorzugsweise geradkettiges oder verzweigtes Alkyl mit je 1 bis 6 Kohlenstoffatomen, besonders bevorzugt mit 1 bis 4 Kohlenstoffatomen und jeweils 1 bis 13, vorzugsweise 1 bis 6, gleichen oder verschiedenen Halogenatomen wie unter Halogen definiert; beispielhaft und vorzugsweise seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluor-methyl, Trifluorchlorethyl, Chlorbutyl, Fluorbutyl.

Unter dem Begriff Alkoxy in den Definitionen von $R^1$ $R^2$, $R^3$ und $R^4$ in den allgemeinen Formeln ist geradkettiges oder verzweigtes Alkoxy mit vorzugsweise 1 bis 6, insbesondere 1 bis 4 Kohlenstoffatomen zu verstehen. Beispielhaft seien Methoxy, Ethoxy, Propoxy, Butoxy sowie ihre Isomeren, i-Propoxy, i-, s- und t-Butoxy genannt.

Unter dem Begriff Alkylthio in den Definitionen von $R^1$, $R^2$, $R^3$ und $R^4$ in den allgemeinen Formeln ist geradkettiges oder verzweigtes Alkylthio mit vorzugsweise 1 bis 6 Kohlenstoffatomen, insbesondere 1 bis 4 Kohlenstoffatome zu verstehen.

Beispielhaft seien Methylthio, Ethylthio, Propylthio, Butylthio, Pentylthio sowie ihre Isomeren, wie z.B. i-Propylthio, i-, s- und t-Butylthio genannt. Besonders bevorzugt sind Methylthio, Ethylthio, Tetrafluorethylthio, Trichlorethylthio, Chlordifluormethylthio und Trifluorchlorethylthio.

Unter dem Begriff Alkoxycarbonyl in den Definitionen von $R^2$ und $R^3$ in den allgemeinen Formeln ist geradkettiges oder verzweigtes Alkoxycarbonyl mit 1 bis 6, vorzugsweise 1 bis 4 Kohlenstoffatomen im Alkoxyrest zu verstehen. Beispielhaft seien genannt: Methoxycarbonyl, Ethoxycarbonyl, n-Propoxycarbonyl, i-Propoxycarbonyl, n-, i-, s- und t-Butoxycarbonyl.

Unsubstituiertes oder substituiertes Aryloxy und Arylthio stehen im allgemeinen in der Definition von $R^1$ in den allgemeinen Formeln für Aryloxy und Arylthio mit vorzugsweise 6 bis 10 Kohlenstoffatomen im Arylteil. Beispielhaft seien unsubstituiertes oder substituiertes Phenoxy oder Phenylthio genannt.

Unsubstituiertes oder substituiertes Aralkyloxy oder Aralkylthio enthalten im allgemeinen in der Definition von $R^1$ vorzugsweise 1 bis 6 Kohlenstoffatome im geradkettigen oder verzweigten Alkylteil und vorzugsweise Phenyl als Arylteil. Als Aralkyloxy- oder -thiogruppen seien beispielhaft Benzyloxy und Benzylthio genannt.

Halogen steht in den Definitionen von $R^1$, $R^2$, $R^3$ und $R^4$ in den allgemeinen Formeln für Fluor, Chlor, Brom, Iod, vorzugsweise für Fluor, Chlor und Brom.

Die Substituenten für die Arylreste bzw. Hetarylreste als solche oder in Zusammensetzungen wie Aryloxy, Arylthio, Aralkyloxy und Aralkylthio umfassen die folgenden Substituenten und können die im folgenden angegebenen Bedeutungen annehmen.

Halogen steht im allgemeinen als Substituent für Fluor, Chlor, Brom und Iod, bevorzugt für Fluor, Chlor und Brom, besonders bevorzugt für Fluor und Chlor.

Alkyl steht im allgemeinen als Substituent oder in Zusammensetzungen wie Phenylalkylthio oder Phenylalkyloxy für geradkettiges oder verzweigtes Alkyl, bevorzugt mit 1 bis 6, besonders bevorzugt mit 1 bis 4 Kohlenstoffatomen, ganz besonders bevorzugt sind Methyl und Ethyl. Die beispielhafte Aufzählung entspricht der weiter oben gegebenen.

Alkoxy steht im allgemeinen als Substituent für geradkettiges oder verzweigtes Alkoxy mit 1 bis 6, besonders bevorzugt 1 bis 4 Kohlenstoffatomen je Alkylrest; beispielhaft und vorzugsweise seien genannt: Methoxy, Ethoxy, n- und i-Propoxy, n-, i-, s- und t-Butoxy, n-Hexoxy und i-Hexoxy.

Alkylthio steht im allgemeinen als Substituent in den Resten für geradkettiges oder verzweigtes Alkylthio mit vorzugsweise 1 bis 6 Kohlenstoffatomen, beispielsweise sind darunter die folgenden Gruppen zu verstehen: Methylthio, Ethylthio, Propylthio, Butylthio, Pentylthio sowie ihre Isomeren, wie z.B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Alkoxycarbonyl steht im allgemeinen als Substituent in den Resten für geradkettiges oder verzweigtes Alkoxycarbonyl mit 1 bis 6, vorzugsweise 1 bis 4 Kohlenstoffatomen im Alkoxyrest; beispielhaft seien genannt: Methoxycarbonyl, Ethoxycarbonyl, n-Propoxycarbonyl, i-Propoxycarbonyl, n-, i-, s- und t-Butoxycarbonyl.

Cycloalkyl steht im allgemeinen als Substituent in den Resten für Cycloalkyl mit vorzugsweise 3 bis 7, insbesondere 3, 5 oder 6 Kohlenstoffatomen. Beispielhaft seien unsubstituiertes oder substituiertes Cyclopropyl, Cyclobutyl, Cyclopentyl, Cyclohexyl und Cycloheptyl genannt.

Alkenyl und Alkinyl stehen im allgemeinen als Substituenten in den Resten für geradkettiges oder verzweigtes Alkenyl oder Alkinyl mit vorzugsweise 2 bis 6, besonders bevorzugt 2 bis 4 und ganz besonders bevorzugt 2 oder 3 Kohlenstoffatomen. Beispielhaft seien Vinyl, Allyl, Propenyl-(2)-, 1-Butenyl, 2-Butenyl, 3-Butenyl, Ethinyl, 1-Propinyl, 2-Propinyl, 1-Butinyl, 2-Butinyl, 3-Butinyl genannt.

Alkenyloxy und Alkinyloxy stehen im allgemeinen als Substituenten in den Resten für geradkettiges oder verzweigtes Alkenyloxy oder Alkinyloxy mit vorzugsweise 2 bis 6, besonders bevorzugt 2 bis 4 und ganz besonders bevorzugt mit 3 Kohlenstoffatomen. Beispielhaft seien Alkyloxy, 2-Propenyloxy, 2-Butenyloxy, 2-Propinyloxy, 2-Butinyloxy genannt.

Halogenalkyl und Halogenalkoxy stehen im allgemeinen als Substituenten in den Resten für geradkettiges oder verzweigtes Halogenalkyl oder Halogenalkoxy mit je 1 bis 6 Kohlenstoffatomen, besonders bevorzugt mit 1 bis 4 Kohlenstoffatomen und jeweils 1 bis 13, vorzugsweise 1 bis 9, gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Halogenalkylthio steht im allgemeinen als Substituent in den Resten für geradkettige oder verzweigtes Halogenalkylthio mit je 1 bis 6 Kohlenstoffatomen, besonders bevorzugt mit 1 bis 4 Kohlenstoffatomen und

jeweils 1 bis 13, vorzugsweise 1 bis 9, gleichen oder verschiedenen Halogenatomen wie unter Halogen definiert; beispielhaft seien genannt: Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

(Di)alkylamino steht im allgemeinen als Substituent in den Resten für eine Aminogruppe mit 1 oder 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n- und i-Propyl genannt seien. Beispielhaft seien Methylamino, Ethylamino, n-Propylamino, i-Propylamino, Dimethylamino, Diethylamino, Di-n-propylamino und Di-i-propylamino aufgeführt.

Wenn im Falle von Dialkylamino die beiden Reste gemeinsam mit dem Stickstoffatom, an welches sie gebunden sind, einen Ring bilden, enthält dieser 5 bis 7, vorzugsweise 5 oder 6 Ringglieder. Der Ring kann 1 bis 3 Doppelbindungen enthalten, vorzugsweise ist er jedoch gesättigt. Der Ring kann ein weiteres Heteroatom, vorzugsweise Sauerstoff, Schwefel oder Stickstoff enthalten und durch 1 oder 2 Alkylgruppen vorzugsweise durch eine Alkylgruppe substituiert sein. Beispielhaft seien genannt: Pyrrolidino, Piperidino, Morpholino, N-Methylpiperazino, Thiomorpholino und 2,6-Dimethylmorpholino.

(Di)alkylaminocarbonyl steht im allgemeinen als Substituent in den Resten für eine Aminocarbonylgruppe mit 1 oder 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n- und i-Propyl genannt seien. Beispielhaft seien Methylaminocarbonyl, Ethylaminocarbonyl, Dimethylaminocarbonyl und Diethylaminocarbonyl genannt.

Wenn im Falle von Dialkylamino die beiden Reste gemeinsam mit dem Stickstoffatom, an welches sie gebunden sind, einen Ring bilden, enthält dieser 5 bis 7, vorzugsweise 5 oder 6 Ringglieder. Der Ring kann 1 bis 3 Doppelbindungen enthalten, vorzugsweise ist er jedoch gesättigt. Der Ring kann ein weiteres Heteroatom, vorzugsweise Sauerstoff, Schwefel oder Stickstoff enthalten und durch 1 oder 2 Alkylgruppen vorzugsweise durch eine Alkylgruppe substituiert sein. Beispielhaft seien genannt: Pyrrolidino, Piperidino, Morpholino, N-Methylpiperazino, Thiomorpholino und 2,6-Dimethylmorpholino.

Alkylcarbonylamino steht im allgemeinen als Substituent in den Resten für geradkettiges oder verzweigtes Alkylcarbonylamino mit 1 bis 6, vorzugsweise 1 bis 4 Kohlenstoffatomen im Alkylrest; beispielhaft seien genannt: Methylcarbonylamino, Ethylcarbonylamino, n-Propylcarbonylamino, i-Propylcarbonylamlno.

Alkylhydrazo steht im allgemeinen als Substituent in den Resten für geradkettiges oder verzweigtes Alkylhydrazo mit 1 bis 6, vorzugsweise 1 bis 4 Kohlenstoffatomen im Alkylteil. Beispielhaft seien genannt: 2-Methylhydrazo, 2-Ethylhydrazo, 2-Propylhydrazo und 2,2-Dimethylhydrazo.

Die erfindungsgemäß zu verwendenden Pyridinylpyrimidin-Derivate sind durch die Formel (I) allgemein definiert. Bevorzugt verwendet werden Verbindungen der Formel (I), bei welchen

$R^1$ für Wasserstoff, Halogen, jeweils geradkettiges oder verzweigtes Alkyl, Alkoxy oder Alkylthio mit jeweils 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen , Amino, Alkyl- oder Dialkylamino mit jeweils 1 bis 6 Kohlenstoffatomen in den jeweiligen geradkettigen oder verzweigten Alkylteilen steht, wobei im Falle von Dialkylamino die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist; weiterhin für jeweils gegebenenfalls einfach oder mehrfach, gleich oder verschieden substituiertes Phenoxy oder Phenylthio, steht, wobei als Phenylsubstituenten genannt seien: Halogen, Cyano, Nitro, Alkyl, Alkoxy oder Alkylthio mit jeweils 1 oder 2 Kohlenstoffatomen, sowie Halogenalkyl, Halogenalkoxy oder Halogenalkylthio mit jeweils 1 oder 2 Kohlenstoffatomen und jeweils 1 bis 5 Fluor- und/oder Chloratomen oder $R^1$ für jeweils gegebenenfalls einfach oder mehrfach, gleich oder verschieden substituiertes Phenylalkoxy oder Phenylalkylthio mit jeweils 1 bis 4 Kohlenstoffatomen im jeweiligen, geradkettigen oder verzweigten Alkylteil steht, wobei als Substituenten des Phenylteils die oben genannten Phenylsubstituenten infrage kommen,

$R^2$ und $R^3$ unabhängig voneinander, gleich oder verschieden, jeweils für Wasserstoff, Halogen, geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils geradkettiges oder verzweigtes Alkoxy oder

Alkylthio mit jeweils 1 bis 6 Kohlenstoffatomen, oder für geradkettiges oder verzweigtes Alkoxycarbonyl mit 1 bis 6 Kohlenstoffatomen im Alkoxyteil stehen, oder

$R^2$ und $R^3$ gemeinsam für eine Alkylenkette mit 3 bis 5 Koh-lenstoffatomen stehen, die über die Ringpositionen 3 und 4 oder 4 und 5 verbunden ist,

$R^4$ für Wasserstoff, Halogen, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, Hydroxy, Mercapto, jeweils geradkettiges oder verzweigtes Alkoxy oder Alkylthio mit jeweils 1 bis 6 Kohlenstoffatomen, Amino, Alkyl- oder Dialkylamino mit jeweils 1 bis 6 Kohlenstoffatomen in den jeweiligen, geradkettigen oder verzweigten Alkylteilen, wobei im Falle von Dialkylamino die beiden Reste gegebenenfalls, gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist, oder für jeweils geradkettiges oder verzweigtes Alkenyloxy oder Alkinyloxy mit jeweils 2 bis 6 Kohlenstoffatomen steht,

$R^5$ für Wasserstoff, geradkettiges oder verzweigtes Alkyl mit 1 bis 6 Kohlenstoffatomen steht und

Ar für gegebenenfalls einfach oder mehrfach, gleich oder verschieden substituiertes Phenyl oder Hetaryl steht, wobei Hetaryl für gegebenenfalls einfach oder mehrfach, gleich oder verschieden substituiertes Hetaryl mit 1 bis 5 Kohlenstoffatomen und 1 bis 4 Heteroatomen aus der Reihe Sauerstoff, Schwefel, Stickstoff steht und an welches gegebenenfalls ein 5- bis 6-gliedriger, gesättigter oder ungesättigter Carbocyclus anelliert ist, steht, wobei als Substituenten genannt seien:

Halogen, Cyano, Nitro, Hydroxy, Amino, Mercapto, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, jeweils geradkettiges oder verzweigtes Alkoxy oder Alkylthio mit jeweils 1 bis 6 Kohlenstoffatomen, jeweils geradkettiges oder verzweigtes Halogenalkylthio oder Halogenalkoxy, mit jeweils 1 bis 6 Kohlenstoffatomen und jeweils 1 bis 13 gleichen oder verschiedenen Halogenatomen, jeweils geradkettiges oder verzweigtes Alkenyl oder Alkinyl mit 2 bis 6 Kohlenstoffatomen, jeweils geradkettiges oder verzweigtes Alkenyloxy oder Alkinyloxy mit 2 bis 6 Kohlenstoffatomen, Cycloalkyl mit 3 bis 8 Kohlenstoffatomen, Phenyl, Phenoxy, Phenylthio, Phenylalkylthio oder Phenylalkyloxy mit jeweils 1 bis 4 Kohlenstoffatomen im geradkettigen oder verzweigten Alkylteil, weiterhin seien als Phenylsubstituenten genannt: (Di)alkylamino mit 1 bis 6 Kohlenstoffatomen in den jeweiligen geradkettigen oder verzweigten Alkylteilen, wobei im Falle von Dialkylamino die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist, oder weiterhin Phenylamino, gegebenenfalls 1- oder 2-fach durch geradkettiges oder verzweigtes Alkyl mit 1 bis 6 Kohlenstoffatomen oder Phenyl substituiertes Hydrazino, Alkyl- oder Phenylcarbonylamino mit 1 bis 6 Kohlenstoffatomen im geradkettigen oder verzweigten Alkylteil, Aminocarbonyl, (Di)alkylaminocarbonyl mit 1 bis 6 Kohlenstoffatomen in den jeweiligen geradkettigen oder verzweigten Alkylteilen wobei im Falle von Dialkylaminocarbonyl die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel-oder Stickstoffatom enthält und welcher gegebenenfalls durch 1 oder 2 Alkylgruppen mit 1 oder 2 Kohlenstoffatomen substituiert ist sowie geradkettiges oder verzweigtes Alkoxycarbonyl mit 1 bis 6 Kohlenstoffatomen, oder zwei benachbarte Phenylsubstituenten stehen gemeinsam für Alkandiyl mit 1 bis 6 Kohlenstoffatomen, welches gegebenenfalls ein oder mehrere Heteroatome aus der Reihe Sauerstoff, Schwefel und Stickstoff enthält.

Besonders bevorzugt verwendet werden Verbindungen der Formel (I), bei welchen

$R^1$ für Wasserstoff, Fluor, Chlor, Brom, jeweils geradkettiges oder verzweigtes Alkyl, Alkoxy oder Alkylthio mit jeweils 1 bis 4 Kohlenstoffatomen, für geradkettiges oder verzweigtes Halogenalkyl mit 1 bis 4 Kohlenstoffatomen und 1 bis 9 Fluor-und/oder Chloratomen für

Amino, Alkyl- oder Dialkylamino mit jeweils 1 bis 4 Kohlenstoffatomen in den jeweiligen geradkettigen oder verzweigten Alkylteilen steht, wobei im Falle von Dialkylamino die beiden Reste gegebenenfalls, gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist, weiterhin für jeweils gegebenenfalls ein- oder zweifach, gleich oder verschieden substituiertes Phenoxy oder Phenylthio, wobei als Phenylsubstituenten genannt seien: Fluor, Chlor, Brom, Cyano, Nitro, Methyl, Trifluormethyl, Methoxy, Trifluormethoxy, Methylthio oder Trifluormethylthio, oder $R^1$ für jeweils gegebenenfalls ein- oder zweifach, gleich oder verschieden substituiertes Phenylalkoxy oder Phenylalkylthio mit jeweils 1 oder 2 Kohlenstoffatomen im Alkylteil steht, wobei als Substituenten des Phenylteils die oben genannten Phenylsubstituenten infrage kommen,

$R^2$ für Wasserstoff, Fluor, Chlor, Brom, für geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, für jeweils geradkettiges oder verzweigtes Alkoxy oder Alkylthio mit 1 bis 4 Kohlenstoffatomen, oder für geradkettiges oder verzweigtes Alkoxycarbonyl mit 1 bis 4 Kohlenstoffatomen im Alkoxyteil steht,

$R^3$ für Wasserstoff, Fluor, Chlor, Brom, geradkettiges oder verzweigtes Alkyl mit 1 bis 4 Kohlenstoffatomen steht oder

$R^2$ und $R^3$ gemeinsam für eine Alkylenkette mit 3 oder 4 Kohlenstoffatomen stehen, die über die Ringpositionen 3 und 4 oder 4 und 5 verbunden ist,

$R^4$ für Wasserstoff, Fluor, Chlor, Brom, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, Hydroxy, Mercapto, jeweils geradkettiges oder verzweigtes Alkoxy oder Alkylthio mit 1 bis 4 Kohlenstoffatomen, Amino, Alkyl- oder Dialkylamino mit jeweils 1 bis 4 Kohlenstoffatomen in den jeweiligen geradkettigen oder verzweigten Alkylteilen, wobei im Falle von Dialkylamino die beiden Reste gegebenenfalls gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist, oder für jeweils geradkettiges oder verzweigtes Alkenyloxy oder Alkinyloxy mit jeweils 3 bis 4 Kohlenstoffatomen steht,

$R^5$ für Wasserstoff, geradkettiges oder verzweigtes Alkyl mit 1 bis 4 Kohlenstoffatomen steht und

Ar für gegebenenfalls 1- bis 3-fach, gleich oder verschieden substituiertes Phenyl steht, wobei als Substituenten genannt seien: Fluor, Chlor, Brom, Iod, Cyano, Nitro, Hydroxy, Mercapto, Amino, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, jeweils geradkettiges oder verzweigtes Alkoxy oder Alkylthio mit jeweils 1 bis 4 Kohlenstoffatomen, jeweils geradkettiges oder verzweigtes Halogenalkoxy oder Halogenalkylthio mit jeweils 1 bis 4 Kohlenstoffatomen und jeweils 1 bis 9 Fluor-und/oder Chloratomen, jeweils geradkettiges oder verzweigtes Alkenyl oder Alkinyl mit jeweils 2 bis 4 Kohlenstoffatomen, jeweils geradkettiges oder verzweigtes Alkenyloxy oder Alkinyloxy mit jeweils 3 bis 4 Kohlenstoffatomen, Cycloalkyl mit 3 bis 6 Kohlenstoffatomen, Phenyl, Phenoxy, Phenylthio, Phenylalkoxy oder Phenylalkylthio mit jeweils 1 oder 2 Kohlenstoffatomen im Alkylteil; weiterhin seien als Phenylsubstituenten genannt: (Di)alkylamino mit 1 bis 4 Kohlenstoffatomen in den jeweiligen geradkettigen oder verzweigten Alkylteilen, wobei im Falle von Dialkylamino die beiden Reste gegebenenfalls, gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist; weiterhin Phenylamino, gegebenenfalls 1- oder 2-fach durch geradkettiges oder verzweigtes Alkyl mit 1 bis 4 Kohlenstoffatomen oder Phenyl substituiertes Hydrazino, Alkyl- oder Phenylcarbonylamino mit 1 bis 4 Kohlenstoffatomen im geradkettigen oder verzweigten Alkylteil, Aminocarbonyl, (Di-)alkylaminocarbonyl mit 1 bis 4 Kohlenstoffatomen in den jeweiligen geradkettigen oder verzweigten Alkylteilen, wobei im Falle von Dialkylaminocarbonyl die beiden Reste gegebenenfalls, gemeinsam mit dem Stickstoffatom, an welches sie gebunden sind, einen 5- oder 6-gliedrigen, gesättigten Ring bilden,

welcher gegebenenfalls ein Sauerstoff-, Schwefel- oder Stickstoffatom enthält und welcher gegebenenfalls durch 1 oder 2 Methylgruppen substituiert ist; sowie geradkettiges oder verzweigtes Alkoxycarbonyl mit 1 bis 4 Kohlenstoffatomen oder zwei benachbarte Phenylsubstituenten stehen gemeinsam für Alkandiyl mit 1 bis 4 Kohlenstoffatomen, welches gegebenenfalls 1 oder 2 Heteroatome aus der Reihe Sauerstoff, Schwefel und Stickstoff enthält;

und

Ar weiterhin für Hetaryl steht, wobei Hetaryl für jeweils gegebenenfalls 1- bis 3-fach, gleich oder verschieden substituiertes Pyridyl, Pyrimidyl, Pyrazinyl, Chinolyl, Isochinolyl, Pyrrolyl, Indolyl, Pyrazolyl, Imidazolyl, Benzimidazolyl, Triazolyl, Thienyl, Benzothienyl, Furyl, Benzofuryl, Thiazolyl, Benzothiazolyl, Isothiazolyl, Oxazolyl, Benzoxazolyl, Isoxazolyl, Thiadiazolyl, Isothiadiazolyl, Oxadiazolyl oder Isoxadiazolyl steht, wobei als Hetarylsubstituenten genannt seien: Fluor, Chlor, Brom, Iod, Cyano, Nitro, Hydroxy, Mercapto, Amino, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, jeweils geradkettiges oder verzweigtes Alkoxy oder Alkylthio mit jeweils 1 bis 4 Kohlenstoffatomen, jeweils geradkettiges oder verzweigtes Halogenalkoxy oder Halogenalkylthio mit jeweils 1 bis 4 Kohlenstoffatomen und jeweils 1 bis 9 Fluor- und/oder Chloratomen, jeweils geradkettiges oder verzweigtes Alkenyl oder Alkinyl mit jeweils 2 bis 4 Kohlenstoffatomen, jeweils geradkettiges oder verzweigtes Alkenyloxy oder Alkinyloxy mit jeweils 3 bis 4 Kohlenstoffatomen, Cycloalkyl mit 3 bis 6 Kohlenstoffatomen, Phenyl, Phenoxy, Phenylthio, Phenylalkoxy oder Phenylalkylthio mit jeweils 1 oder 2 Kohlenstoffatomen im Alkylteil.

Ganz besonders bevorzugt verwendet werden Verbindungen der Formel (I), bei welchen

$R^1$ für Wasserstoff, Fluor, Chlor, Methyl, Methoxy, Ethoxy, n-Propoxy oder i-Propoxy, Methylthio, Ethylthio, Trifluormethyl, Trichlormethyl, Difluormethyl oder Chlorfluormethyl, für Methylamino, Dimethylamino, Ethylamino, Diethylamino, Pyrrolidino, Piperidino, Morpholino, Thiomorpholino, N-Methyl-piperazino, 2,6-Dimethylmorpholino, für Phenoxy, Phenylthio, Benzyloxy oder Benzylthio steht,

$R^2$ für Wasserstoff, Fluor, Chlor, Brom, Methyl, Ethyl, n-Propyl oder i-Propyl, für Trifluormethyl, Trichlormethyl, Difluormethyl, Dichlormethyl, für Methoxy oder Ethoxy, für Methylthio, für Methoxy- oder Ethoxycarbonyl steht,

$R^3$ für Wasserstoff, Fluor, Chlor, Brom, Methyl, Ethyl, n-Propyl, i-Propyl oder t-Butyl steht,

$R^4$ für Wasserstoff, Fluor, Chlor, Brom, Methyl, Trifluormethyl, Trichlormethyl, Hydroxy, Mercapto, Methoxy, Ethoxy, n-Propoxy, i-Propoxy, Methylthio, Ethylthio, n-Propylthio oder i-Propylthio, n-Butylthio, i-Butylthio, s-Butylthio oder t-Butylthio, für Amino, Methylamino, Ethylamino, Dimethylamino, Diethylamino, Pyrrolidino, Piperidino, Morpholino, Thiomorpholino, N-Methylpiperazino oder 2,6-Dimethylmorpholino, für 2-Propenyloxy oder 2-Propinyloxy steht,

$R^5$ für Wasserstoff, Methyl, Ethyl, n-Propyl, i-Propyl oder n-Butyl steht und

Ar für gegebenenfalls 1- bis 3-fach, gleich oder verschieden substituiertes Phenyl steht, wobei als Substituenten genannt seien:

Fluor, Chlor, Brom, Iod, Cyano, Nitro, Hydroxy, Amino, Mercapto, Methyl, Ethyl, n-Propyl oder i-Propyl, n-Butyl, s-Butyl, i-Butyl oder t-Butyl, Trifluormethyl, Trichlormethyl, Difluormethyl, Methoxy, Ethoxy, Methylthio, Ethylthio, Trifluormethoxy, Difluormethoxy, Trifluormethylthio, Chlordifluormethylthio, Vinyl, 2-Propenyl, 2-Propinyl, 2-Propenyloxy, 2-Propinyloxy, Cyclopropyl, Cyclopentyl, Cyclohexyl, Phenyl, Phenoxy, Phenylthio, Phenylmethoxy oder Phenylmethylthio, weiterhin seien als Phenylsubstituenten genannt:

Methylamino, Dimethylamino, Ethylamino, Diethylamino, Pyrrolidino, Piperidino, Morpholino, Thiomorpholino, N-Methylpiperazino, 2,6-Dimethylmorpholino, Phenylamino, Hydrazino, 2-Methylhydrazo, 2,2-Dimethylhydrazo, 2-Phenylhydrazo, Methylcarbonylamino, Phenylcarbonylamino, Aminocarbonyl, Methylaminocarbonyl, Dimethylaminocarbonyl, Methoxycarbonyl oder Ethoxycarbonyl; und weiterhin für jeweils gegebenenfalls 1- bis 3-fach, gleich oder verschieden substituiertes Pyridyl, Pyrimidyl, Pyrazinyl, Chinolyl, Pyrrolyl, Indolyl, Pyrazolyl, Imidazolyl, Benzimidazolyl, Thienyl, Benzothienyl, Furyl, Benzofuryl, Thiazolyl, Oxazolyl, Thiadiazolyl oder Oxadiazolyl steht, wobei als Hetarylsubstituenten genannt seien:

Fluor, Chlor, Brom, Iod, Cyano, Nitro, Hydroxy, Amino, Mercapto, Methyl, Ethyl, n-Propyl oder i-Propyl, n-Butyl, s-Butyl, i-Butyl oder t-Butyl, Trifluormethyl, Trichlormethyl, Difluormethyl, Methoxy, Ethoxy, Methylthio, Ethylthio, Trifluormethoxy, Difluormethoxy, Trifluormethylthio, Chlordifluormethylthio, Vinyl, 2-Propenyl, 2-Propinyl, 2-Propenyloxy, 2-Propinyloxy, Cyclopropyl, Cyclopentyl, Cyclohexyl, Phenyl, Phenoxy, Phenylthio, Phenylmethoxy oder Phenylmethylthio, weiterhin seien als Hetarylsubstituenten genannt:
Methylamino, Dimethylamino, Ethylamino, Diethylamino, Pyrrolidino, Piperidino, Morpholino, Thiomorpholino, N-Methylpiperazino, 2,6-Dimethylmorpholino, Phenylamino, Hydrazino, 2-Methylhydrazo, 2,2-Dimethylhydrazo, 2-Phenylhydrazo, Methylcarbonylamino, Phenylcarbonylamino, Aminocarbonyl, Methylaminocarbonyl, Dimethylaminocarbonyl, Methoxycarbonyl oder Ethoxycarbonyl.

Im einzelnen seien die folgenden Pyridinylpyrimidin-Derivate der allgemeinen Formel (I) genannt:

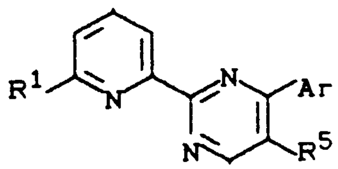

(I)

$R^2$, $R^3$, $R^4$ = H; Ph = Phenyl.

| Verb.-Nr. | $R_1$ | $R_5$ | Ar | physikal. Daten |
|---|---|---|---|---|
| 1 | H | H | Ph | $n_D^{20}$ = 1.6605 |
| 2 | H | H | $3-CH_3-Ph$ | Öl |
| 3 | H | H | $4-CH_3-Ph$ | Fp. 137° C |
| 4 | H | H | 3-Cl-Ph | Öl |
| 5 | H | H | $4-CH_3O-Ph$ | Öl |
| 6 | $CH_3$ | H | $2-CH_3-Ph$ | Öl |
| 7 | H | $n-C_3H_7$ | Ph | Fp. 101° C |
| 8 | H | H | 2-Cl-Ph | Fp. 100° C |
| 9 | H | H | 4-Cl-Ph | Fp. 109° C |
| 10 | H | H | 4-Br-Ph | Öl |
| 11 | H | H | α-Naphthyl | Öl |
| 12 | H | H | 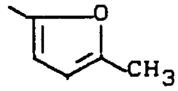 | Fp. 66° C |
| 13 | H | H | 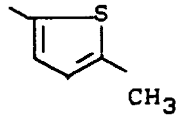 | Fp. 60° C |
| 14 | H | H | 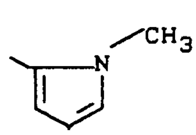 | Fp. 64° C |

Die erfindungsgemäß zu verwendenden Verbindungen der Formel (I) kann man nach einem der im folgenden beschriebenen Verfahren erhalten.

A) Man erhält Pyridinylpyrimidin-Derivate der Formel (Ia)

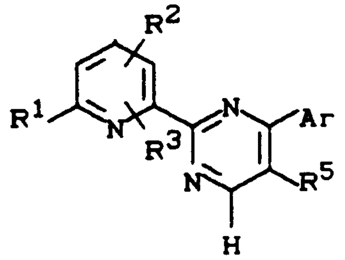

(Ia)

in welcher

$R^1$, $R^2$, $R^3$, $R^5$ und Ar die oben angegebene Bedeutung haben,

wenn man halogensubstituierte Pyridinylpyrimidin-Derivate der Formel (II)

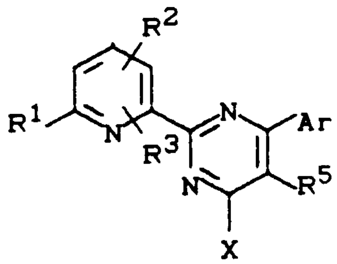

(II)

in welcher

$R^1$, $R^2$, $R^3$, $R^5$ und Ar die oben angegebene Bedeutung haben, und

X für Halogen steht,

gegebenenfalls in Gegenwart eines Lösungsmittels, gegebenenfalls in Gegenwart einer Base, in Gegenwart eines Katalysators und unter Wasserstoffdruck enthalogeniert,

oder

B) man erhält Pyridinylpyrimidin-Derivate der Formel (Ib)

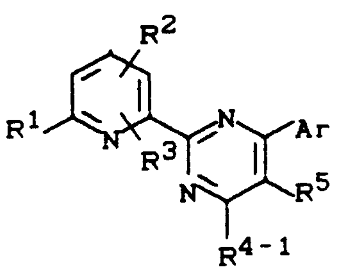

(Ib)

in welcher

$R^1$, $R^2$, $R^3$, $R^5$ und Ar die oben angegebene Bedeutung haben, und

$R^{4-1}$ für Mercapto, Alkoxy, Alkylthio, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist oder für Alkenyloxy oder Alkinyloxy steht,

wenn man halogensubstituierte Pyridinylpyrimidin-Derivate der Formel (II)

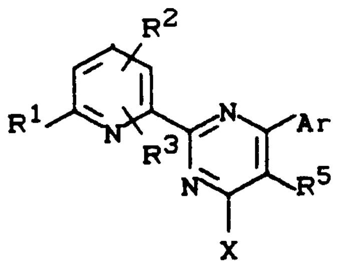

(II)

in welcher

$R^1$, $R^2$, $R^3$, $R^5$ und Ar die oben angegebene Bedeutung haben und

X für Halogen steht,

mit Verbindungen der Formel (III)

$R^{4-1}$-M (III)

in welcher

$R^{4-1}$ die oben angegebene Bedeutung hat und

M für Wasserstoff oder für ein Alkalimetallkation steht,

gegebenenfalls in Gegenwart eines Verdünnungsmittels und gegebenenfalls in Gegenwart einer Base umsetzt,

oder

C) man erhält Pyridinylpyrimidin-Derivate der Formel (Ic)

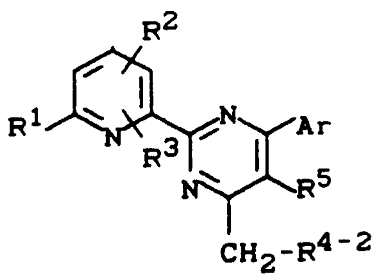

(Ic)

in welcher

$R^1$, $R^2$, $R^3$, $R^5$ und Ar die oben angegebene Bedeutung haben und

$R^{4-2}$ für Wasserstoff oder Alkyl steht,

wenn man halogensubstituierte Pyridinylpyrimidin-Derivate der Formel (II)

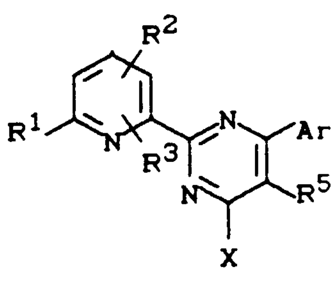

(II)

in welcher

$R^1$, $R^2$, $R^3$, $R^5$ und Ar die oben angegebene Bedeutung haben und

X für Halogen steht,

mit Diester-Derivaten der Formel (IV)

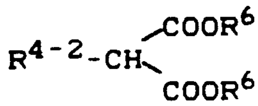

(IV)

in welcher

$R^{4-2}$ die oben angegebene Bedeutung hat und

$R^6$ für Methyl oder Ethyl steht,

gegebenenfalls in Gegenwart eines Verdünnungsmittels und gegebenenfalls in Gegenwart einer Base umsetzt und die so erhaltenen substituierten Pyridinylpyrimidine gegebenenfalls in Gegenwart eines Verdünnungsmittels und in Gegenwart einer Base hydrolysiert und anschließend in Gegenwart einer Säure decarboxyliert;

oder

D) man erhält die Pyridinylpyrimidin-Derivate der Formel (Id)

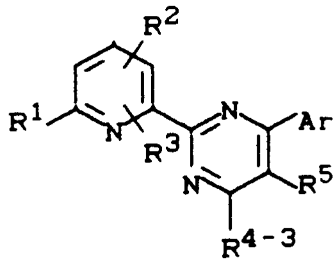

(Id)

in welcher

$R^1$, $R^2$, $R^3$, $R^5$ und Ar die oben angegebene Bedeutung haben und

$R^{4-3}$ für Wasserstoff, Alkyl oder Halogenalkyl steht,

wenn man Picolinamidin-Derivate der Formel (V)

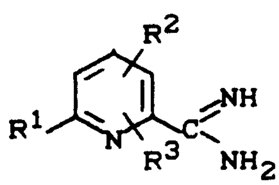

(V)

in welcher

$R^1$, $R^2$ und $R^3$ die oben angegebene Bedeutung haben, oder deren Säureadditionssalze

α) mit Arylketonen der Formel (VI)

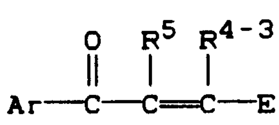

(VI)

in welcher

$R^5$ und Ar die oben angegebene Bedeutung haben,

$R^{4-3}$ für Wasserstoff, Alkyl oder Halogenalkyl steht und

E für eine Abgangsgruppe steht,

gegebenenfalls in Gegenwart eines Verdünnungsmittels und gegebenenfalls in Gegenwart einer Base cyclisiert,

oder

β) mit Acetalen der Formel (VII)

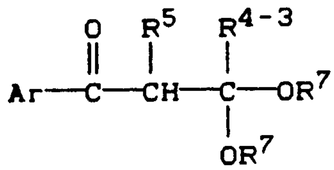

(VII)

in welcher

$R^5$ und Ar die oben angegebene Bedeutung haben,

$R^{4-3}$ für Wasserstoff, Alkyl oder Halogenalkyl steht und

$R^7$ für Alkyl mit 1 bis 4 Kohlenstoffatomen steht,

gegebenenfalls in Gegenwart eines Verdünnungsmittels und gegebenenfalls in Gegenwart einer Base umsetzt,

γ) mit 1,3-Dioxo-Derivaten der Formel (VIII)

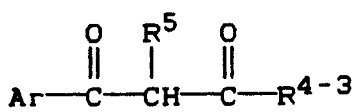

(VIII)

in welcher

$R^5$ und Ar die oben angegebene Bedeutung haben und

$R^{4-3}$ für Wasserstoff, Alkyl oder Halogenalkyl steht,

gegebenenfalls in Gegenwart eines Verdünnungsmittels und in Gegenwart einer Base umsetzt,

oder

δ) mit Diacetalen der Formel (IX)

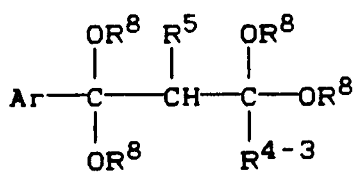

(IX)

in welcher

Ar und $R^5$ die oben angegebene Bedeutung haben,

$R^{4-3}$ für Wasserstoff, Alkyl oder Halogenalkyl steht und

$R^8$ für Alkyl mit 1 bis 4 Kohlenstoffatomen steht,

gegebenenfalls in Gegenwart eines Verdünnungsmittels umsetzt.

Verwendet man beispielsweise 4-Chlor-2-(2-pyridyl)-6-(4-fluor-phenyl)-pyrimidin als Ausgangsstoff und Wasserstoff sowie Palladium-Kohle als Katalysator, so läßt sich das Verfahren (A) durch das folgende Formelschema beschreiben:

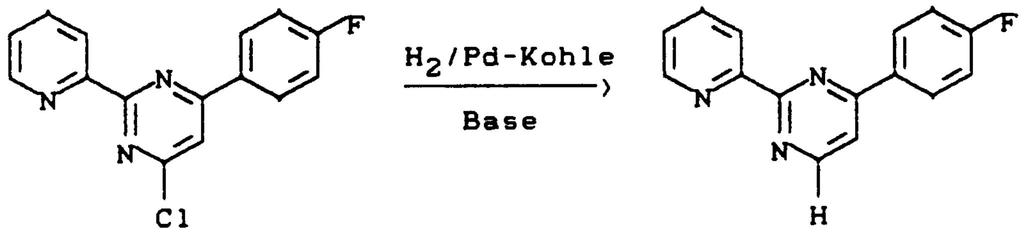


Verwendet man beispielsweise 4-Chlor-2-(2-pyridyl)-6-(4-fluor-phenyl)-pyrimidin und Natriummethanolat als Ausgangsstoffe, so kann das Verfahren (B) durch das folgende Formelschema beschrieben werden:

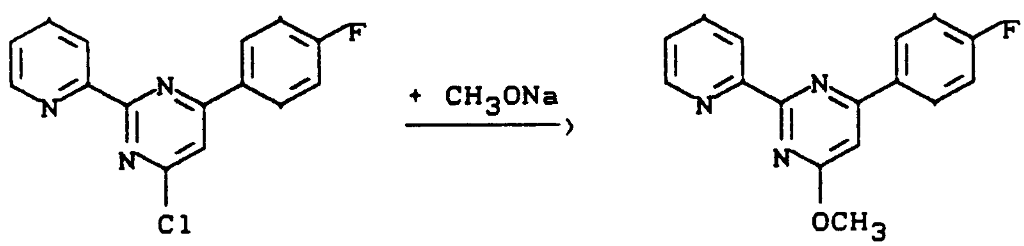


Verwendet man beispielsweise 4-Chlor-2-(2-pyridyl)-6-(4-fluor-phenyl)-pyrimidin und Malonsäurediethylester, so läßt sich das Verfahren (C) durch das folgende Formelschema darstellen:

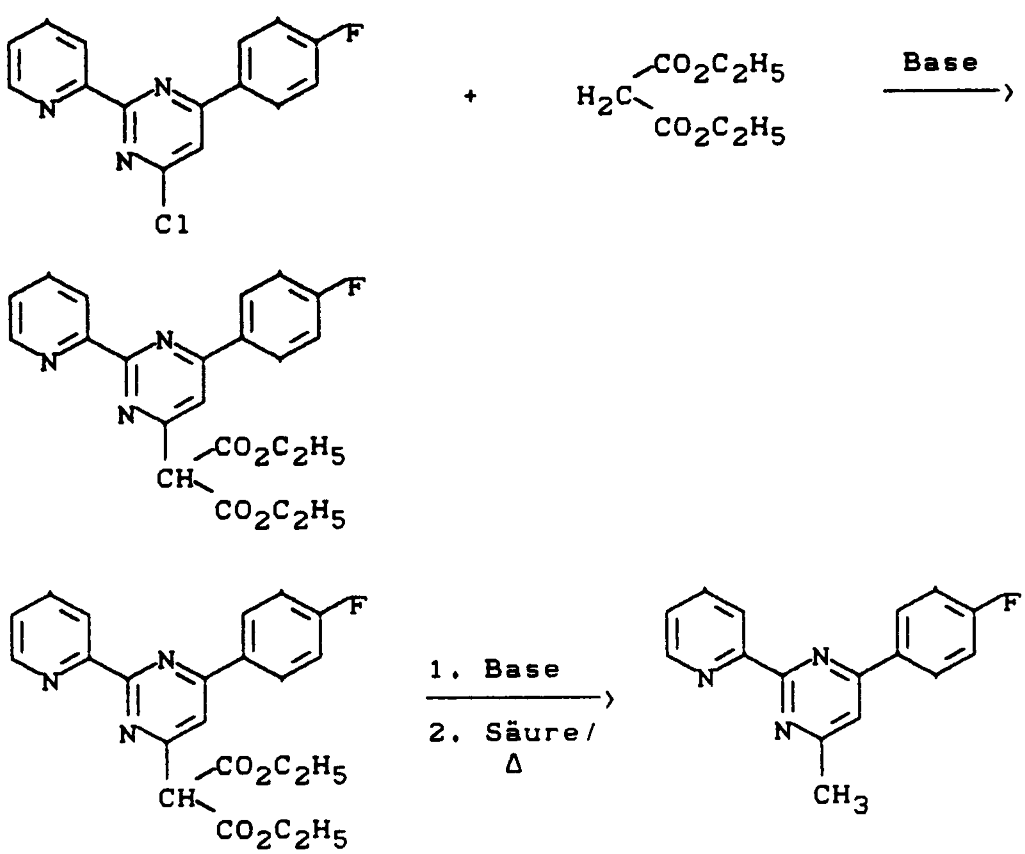


Verwendet man beispielsweise 2-Pyridinyl-amidin-Hydrochlorid und 1-(4-Cyclohexyl-phenyl)-3-dimethylaminopropen-on und Natriummethylat als Ausgangsstoffe, so läßt sich das Verfahren (D-$\alpha$) durch

das folgende Formelschema darstellen:

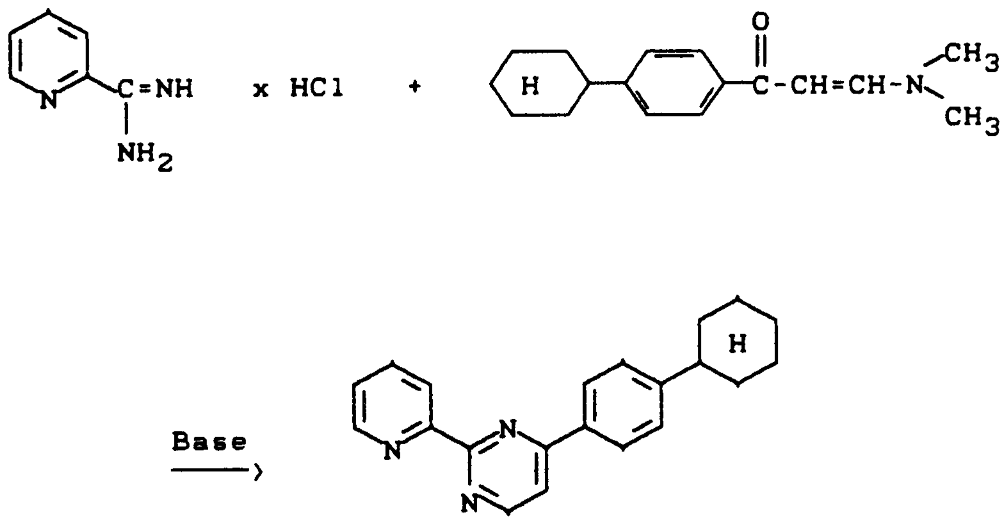


Verwendet man beispielsweise 2-Pyridinyl-amidin-Hydrochlorid und 1-Phenyl-3,3-dimethoxy-propan-1-on, so läßt sich das Verfahren (D-β) durch das folgende Formelschema beschreiben:

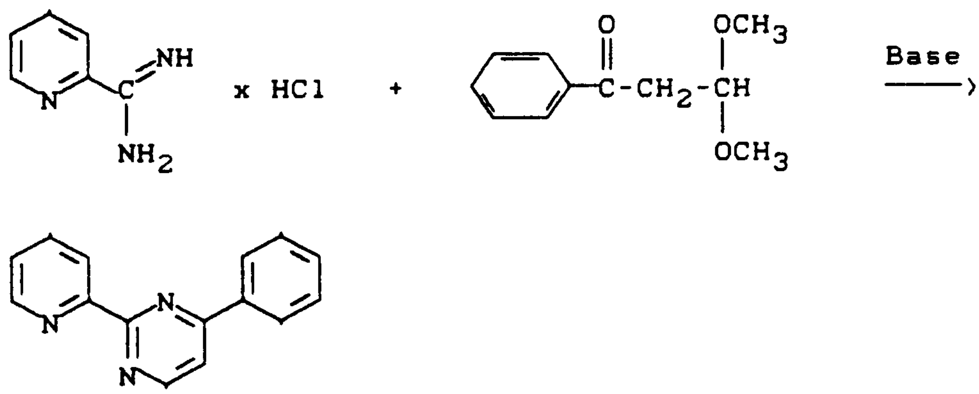


Verwendet man beispielsweise 2-Pyridinyl-amidin-Hydrochlorid und 1-Phenyl-1,3-butandion als Ausgangsstoffe, so läßt sich das Verfahren (D-γ) durch das folgende Formelschema beschreiben:

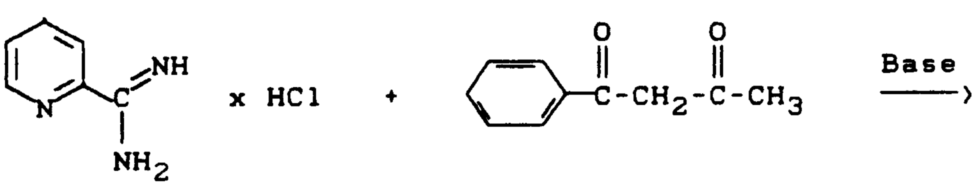

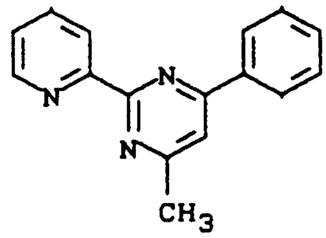

Verwendet man beispielsweise 2-Pyridinyl-amidin-Hydrochlorid und 1,1,3,3-Tetramethoxypropyl-benzol, so läßt sich das Verfahren (D-δ) durch das folgende Formelschema beschreiben:

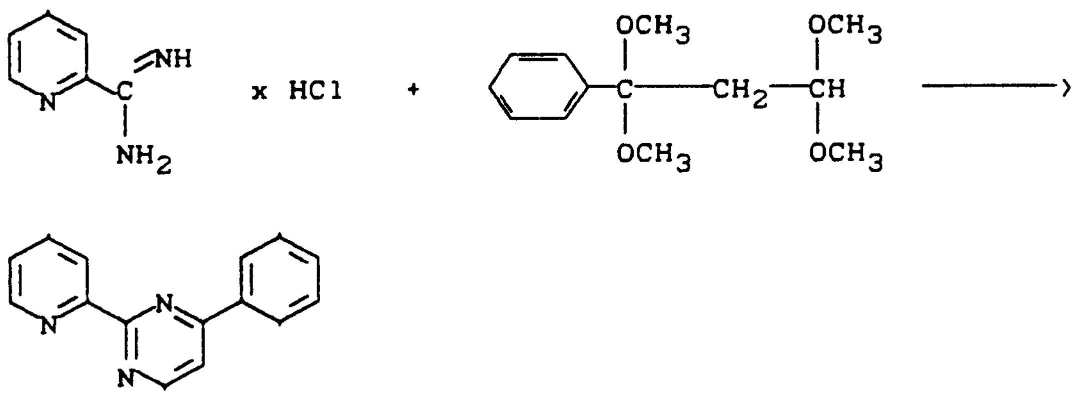


Die zur Durchführung der Verfahren (A), (B) und (C) als Ausgangsstoffe benötigten halogensubstituierten Pyridinylpyrimidin-Derivate sind durch die Formel (II) allgemein definiert. In dieser Formel (II) stehen $R^1$, $R^2$, $R^3$, $R^5$, Ar und X für diejenigen Reste, die bereits im Zusammenhang mit der Beschreibung der erfindungsgemäß zu verwendenden Stoffe der Formel (I) als bevorzugt für diese Substituenten genannt wurden.

Die Verbindungen der Formel (II) sind neu. Sie lassen sich jedoch nach bekannten Verfahren in analoger Weise erhalten, indem man beispielsweise Picolinamin-Derivate der Formel (V)

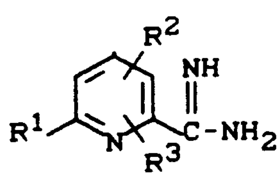 (V)

in welcher

$R^1$, $R^2$ und $R^3$ die oben angegbene Bedeutung haben, oder deren Säureadditionssalze

mit Benzoylessigsäureester-Derivaten der Formel (X)

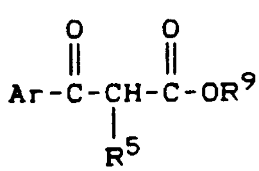 (X)

in welcher

Ar und $R^5$ die oben angegebene Bedeutung haben und
$R^9$ für Methyl oder Ethyl steht,

gegebenenfalls in Gegenwart eines Verdünnungsmittels wie beispielsweise Methanol, Ethanol, Dioxan, Tetrahydrofuran, Pyridin, N,N-Dimethylformamid, Wasser oder einer Mischung aus Wasser und den genannten Verdünnungsmitteln und gegebenenfalls in Gegenwart einer Base, wie beispielsweise Natriumhydroxid, Kaliumhydroxid, Kaliumcarbonat, Natriummethanolat, Kaliummethanolat, Triethylamin oder N,N-Diethylanilin bei Temperaturen von 50 bis 150 °C zu den ebenfalls neuen Verbindungen der Formel (XI)

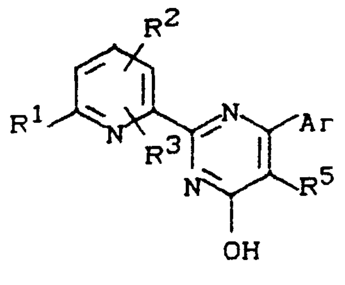

(XI)

in welcher
$R^1$, $R^2$, $R^3$, $R^5$ und Ar die oben angegebene Bedeutung haben,

cyclisiert und die so erhaltenen Verbindungen der Formel (XI) gegebenenfalls nach ihrer Isolierung, gegebenenfalls in Gegenwart eines Verdünnungsmittels, wie beispielsweise Benzol, Toluol oder Chlorbenzol mit einem Halogenierungsreagenz, wie beispielsweise Thionylchlorid, Phosgen, Phosphorylchlorid, Phosphorpentachlorid, Phosphorylbromid oder Phosphortribromid bei Temperaturen zwischen 50 °C und 150 °C halogeniert (vgl. EP-A 259 139 und EP-A 270 362).

Die Picolinamin-Derivate der Formel (V) oder deren Säureadditionssalze sind teilweise bekannt und/oder können in Analogie zu bekannten Verfahren hergestellt werden (vgl. EP-A 259 139 und EP-A 270 362).

Die zur Durchführung des Verfahrens (B) außerdem als Ausgangsstoffe benötigten Verbindungen sind durch die Formel (III) allgemein definiert. In dieser Formel (III) stehen $R^{4-1}$ und M vorzugsweise für diejenigen Reste, die bereits im Zusammenhang mit der Beschreibung der erfindungsgemäß zu verwendenden Stoffe der Formel (I) als bevorzugt für diese Substituenten genannt wurden.

Die Verbindungen der Formel (III) sind allgemein bekannte Verbindungen der organischen Chemie.

Die zur Durchführung des Verfahrens (C) außerdem als Ausgangsstoffe benötigten Diester-Derivate sind durch die Formel (IV) allgemein definiert. In dieser Formel (IV) stehen $R^{4-2}$ und $R^6$ vorzugsweise für diejenigen Reste, die bereits im Zusammenhang mit der Beschreibung der erfindungsgemäß zu verwendenden Stoffe der Formel (I) als bevorzugt für diese Substituenten genannt wurden.

Die Verbindungen der Formel (IV) sind allgemein bekannte Verbindungen der organischen Chemie.

Die zur Durchführung des Verfahrens (D-$\alpha$) außerdem als Ausgangsstoffe benötigten Arylketone sind durch die Formel (VI) allgemein definiert. In dieser Formel (VI) stehen $R^{4-3}$, $R^5$ und Ar für diejenigen Reste, die bereits im Zusammenhang mit der Beschreibung der erfindungsgemäß zu verwendenden Stoffe der Formel (I) als bevorzugt für diese Substituenten genannt wurden.

E steht vorzugsweise für Halogen, insbesondere Chlor, Brom oder Iod, für Dialkylamino, wie beispielsweise Dimethylamino oder für jeweils gegebenenfalls substituiertes Alkylsulfonyloxy, Alkoxysulfonyloxy oder Arylsulfonyloxy, wie beispielsweise Methansulfonyloxy, Trifluormethansulfonyloxy, Methoxysulfonyloxy, Ethoxysulfonyloxy oder p-Toluolsulfonyloxy.

Die Verbindungen der Formel (VI) sind allgemein bekannte Verbindungen der organischen Chemie.

Die zur Durchführung des Verfahrens (D-$\beta$) außerdem als Ausgangsstoffe benötigten Acetale sind durch die Formel (VII) allgemein definiert. In dieser Formel (VII) stehen Ar, $R^{4-3}$ und $R^5$ für diejenigen Reste, die bereits im Zusammenhang mit der Beschreibung der erfindungsgemäß zu verwendenden Stoffe der Formel (I) als bevorzugt für diese Substituenten genannt wurden.

$R^7$ steht vorzugsweise für Methyl oder Ethyl.

Die zur Durchführung des Verfahrens (D-$\gamma$) außerdem benötigten 1,3-Dioxo-Derivate sind durch die Formel (VIII) allgemein definiert. In dieser Formel (VIII) stehen Ar, $R^{4-3}$ und $R^5$ für diejenigen Reste, die bereits im Zusammenhang mit der Beschreibung der erfindungsgemäß zu verwendenden Stoffe der Formel (I) als bevorzugt für diese Substituenten genannt wurden.

Die Verbindungen der Formeln (VII) und (VIII) sind allgemein bekannte Verbindungen der organischen Chemie.

Die zur Durchführung des Verfahrens (D-$\delta$) außerdem als Ausgangsstoffe benötigten Diacetale sind

durch die Formel (IX) allgemein definiert. In dieser Formel (IX) stehen Ar, $R^{4-3}$ und $R^5$ für diejenigen Reste, die bereits im Zusammenhang mit der Beschreibung der erfindungsgemäß zu verwendenden Stoffe der Formel (I) als bevorzugt für diese Substituenten genannt wurden.

$R^8$ steht vorzugsweise für Methyl oder Ethyl.

Die Verbindungen der Formel (IX) sind allgemein bekannte Verbindungen der organischen Chemie.

Das Verfahren (A) zur Herstellung der neuen Verbindungen der Formel (Ia) wird vorzugsweise unter Verwendung von Verdünnungsmitteln durchgeführt. Als Verdünnungsmittel kommen dabei praktisch alle inerten organischen Lösungsmittel in Frage. Hierzu gehören Benzol, Toluol, Xylol, Tetrahydrofuran und Dioxan, Ester wie Essigsäuremethylester und -ethylester, Alkohole, wie Methanol, Ethanol und Isopropanol sowie Wasser. Vorzugsweise verwendet man Wasser, Methanol, Ethanol, Essigsäureethylester und Toluol oder Mischungen dieser Verdünnungsmittel.

Die Reaktionstemperaturen können bei dem Verfahren (A) in einem größeren Bereich variiert werden. Im allgemeinen arbeitet man bei Temperaturen zwischen 0 °C und +100 °C, vorzugsweise bei Temperaturen zwischen +20 °C und +50 °C.

Das Verfahren (A) wird vorzugsweise unter Verwendung von Katalysatoren durchgeführt. Als solche kommen übliche Hydrierungskatalysatoren infrage, wie beispielsweise Raney-Nickel, elementares Platin oder Palladium, vorzugsweise verwendet man Palladium/Aktivkohle.

Das Verfahren (A) wird in Gegenwart von Basen durchgeführt. Als solche kommen beispielsweise Alkalimetallhydroxide wie z.B. Natrium- und Kaliumhydroxid, Erdalkalihydroxide wie z.B. Calciumhydroxid, Alkalicarbonate, -acetate und -alkoholate, wie Natrium- und Kaliumcarbonat, Natrium- und Kaliummethylat bzw. -ethylat, Ammoniak, Amine, wie Diethylamin oder basische Ionenaustauscher infrage.

Zur Durchführung des Verfahrens (A) arbeitet man im allgemeinen bei einem Wasserstoffdruck von 1 bis 5 Atmosphären, vorzugsweise bei 1 bis 3 Atmosphären. Zur Aufarbeitung wird der Katalysator abfiltriert, die Reaktionslösung mit Wasser versetzt und mit einem organischen Lösungsmittel extrahiert. Die weitere Aufarbeitung erfolgt nach üblichen Methoden.

Zur Durchführung des Verfahrens (A) setzt man pro Mol an halogeniertem Pyridinylpyrimidin-Derivat der Formel (II) im allgemeinen 1 bis 10 Mol, vorzugsweise 1 bis 5 Mol an Wasserstoff sowie im allgemeinen 0,01 bis 1 Mol, vorzugsweise 0,01 bis 0,1 Mol an Katalysator ein.

Das Verfahren (B) zur Herstellung der neuen Verbindungen der Formel (Ib) wird vorzugsweise unter Verwendung von Verdünnungsmitteln durchgeführt. Als Verdünnungsmittel kommen dabei praktisch alle inerten organischen Lösungsmittel in Frage. Hierzu gehören aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol oder Isopropanol. Vorzugsweise verwedet man Ether wie Diethylether, Dioxan und Tetrahydrofuran sowie Alkohole wie Methanol und Ethanol oder Mischungen dieser Verdünnungsmittel.

Die Reaktionstemperaturen können bei dem Verfahren (B) in einem größeren Bereich variiert werden. Im allgemeinen arbeitet man bei Temperaturen zwischen 0 °C und +150 °C, vorzugsweise bei Temperaturen zwischen +10 °C und +120 °C.

Das Verfahren (B) wird in Gegenwart von Basen durchgeführt. Vorzugsweise verwendet man Alkalimetallhydroxide wie Natrium- oder Kaliumhydroxid oder organische Basen wie Natriummethanolat, Triethylamin oder Pyridin.

Zur Durchführung des Verfahrens (B) setzt man pro Mol an halogensubstituiertem Pyridinylpyrimidin-Derivat der Formel (II) im allgemeinen 1 bis 3 Mol, vorzugsweise 1 bis 1,5 Mol an Verbindung der Formel (III) ein.

Zur Durchführung des Verfahrens (B) wird das Reaktionsgemisch mehrere Stunden bei der erforderlichen Temperatur gerührt. Die Aufarbeitung erfolgt nach üblichen Methoden, eine anschließende Reinigung erfolgt gegebenenfalls durch Chromatographie.

Das Verfahren (C) zur Herstellung der neuen Verbindungen der Formel (Ic) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Dimethylsulfoxid und Sulfolan sowie Gemische dieser Verdünnungsmittel.

Die Reaktionstemperaturen können beim ersten Reaktionsschritt des Verfahrens (C) in einem größeren

Bereich variiert werden. Im allgemeinen arbeitet man bei Temperaturen zwischen -10 °C und +180 °C, vorzugsweise bei Temperaturen zwischen 0 °C und +150 °C.

Der erste Reaktionsschritt des Verfahrens (C) wird in Gegenwart von Basen durchgeführt. Vorzugsweise verwendet man Alkalimetallhydride wie beispielsweise Natriumhydrid, Alkyllithium wie n-Butyllithium, Lithiumdialkylamide wie z.B. Lithiumdiisopropylamid (LDA), Alkalimetallalkoholate wie z.B. Natriummethanolat und Natriumethanolat und Metallhydroxide wie z.B. Natriumhydroxid.

Zur Durchführung des ersten Reaktionsschrittes des Verfahrens (C) setzt man pro Mol an halogensubstituiertem Pyridinylpyrimidin-Derivat der Formel (II) im allgemeinen 0,5 bis 2 Mol, vorzugsweise 1 bis 1,5 Mol an Diester-Derivat der Formel (IV) sowie im allgemeinen 1 bis 3 Mol, vorzugsweise 1 bis 2 Mol an Base ein.

Die Reaktionstemperaturen können beim zweiten Reaktionsschritt des Verfahrens (C) in einem größeren Bereich variiert werden. Im allgemeinen arbeitet man bei Temperaturen zwischen 0 °C und +150 °C, vorzugsweise bei Temperaturen zwischen +10 °C und +100 °C.

Der zweite Reaktionsschritt des Verfahrens (C) wird in Gegenwart von Basen durchgeführt. Vorzugsweise verwendet man Alkalimetallhydroxide wie z.B. Natriumhydroxid und Alkalimetallcarbonate wie z.B. Natriumcarbonat.

Zur Durchführung des zweiten Reaktionsschrittes des Verfahrens (C) setzt man pro Mol an verwendetem halogensubstituierten Pyridinylpyrimidin-Derivat der Formel (II) im allgemeinen 1 bis 6 Mol, vorzugsweise 2,1 bis 5 Mol an Base ein.

Die Reaktionstemperaturen können bei der Decarboxylierungsreaktion des Verfahrens (C) in einem größeren Bereich variiert werden. Im allgemeinen arbeitet man bei Temperaturen zwischen 0 °C und +200 °C, vorzugsweise bei Temperaturen zwischen +20 °C und +150 °C.

Die Decarboxylierungsreaktion des Verfahrens (C) wird in Gegenwart von Säuren durchgeführt. Vorzugsweise verwendet man anorganische Säuren wie z.B. Schwefelsäure oder Chlorwasserstoffsäure und organische Säuren wie z.B. Essigsäure.

Zur Durchführung der Decarboxylierungsreaktion des Verfahrens (C) setzt man pro Mol an verwendetem halogensubstituiertem Pyridinylpyrimidin der Formel (II) im allgemeinen 1 bis 10 Mol, vorzugsweise 2,5 bis 6 Mol an Säure ein.

Die Durchführung des Verfahrens (C) erfolgt nach üblichen Methoden. Zur Aufarbeitung wird die Reaktionsmischung neutralisiert, konzentriert und extrahiert. Die Reinigung des Produktes erfolgt durch Chromatographie oder Umkristallisation.

Das Verfahren (D-α) zur Herstellung der Verbindungen der Formel (I-d) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise Ether wie Tetrahydrofuran und Dioxan, Amide wie z. B. Dimethylformamid, Dimethylacetamid und Pyridin sowie Alkohole wie Methanol und Ethanol.

Die Reaktionstemperaturen können bei dem Verfahren (D-α) in einem größeren Bereich variiert werden. Im allgemeinen arbeitet man bei Temperaturen zwischen 0 °C und +200 °C, vorzugsweise bei Temperaturen zwischen +50 °C und +150 °C.

Das Verfahren (D-α) wird in Gegenwart von Basen durchgeführt. Vorzugsweise verwendet man Metallalkoholate wie z.B. Natriummethanolat und Natriumethanolat sowie organische Basen wie z.B. Trimethylamin, Triethylamin und N,N-Diethylanilin.

Die Reaktionsdurchführung und Aufarbeitung erfolgt nach üblichen Methoden durch Einengen unter vermindertem Druck und gegebenenfalls anschließender chromatographischer Reinigung.

Zur Durchführung des Verfahrens (D-α) setzt man pro Mol an Picolinamidin-Derivat der Formel (V) im allgemeinen 1 bis 3 Mol, vorzugsweise 1 bis 1,5 Mol an Arylketon der Formel (VI) sowie im allgemeinen 0,1 bis 5 Mol, vorzugsweise 0,1 bis 2,5 Mol an Base ein.

Das Verfahren (D-β) zur Herstellung der Verbindungen der Formel (I-d) wird vorzugsweise unter Verwendung von Verdünnungsmitteln durchgeführt. Als Verdünnungsmittel kommen dabei Ether wie Tetrahydrofuran und Dioxan, Amide wie z. B. Dimethylformamid, Dimethylacetamid sowie Pyridin, Wasser oder Mischungen der genannten Lösungsmittel infrage.

Die Reaktionstemperaturen können bei dem Verfahren (D-β) in einem größeren Bereich variiert werden. Im allgemeinen arbeitet man bei Temperaturen zwischen 0 °C und +200 °C, vorzugsweise bei Temperaturen zwischen +50 °C und +150 °C.

Das Verfahren (D-β) wird in Gegenwart von Basen durchgeführt. Vorzugsweise verwendet man anorganische Basen wie Natrium- oder Kaliumhydroxid oder Kaliumcarbonat oder organische Basen wie Natriummethanolat, Triethylamin oder N,N-Diethylanilin.

Zur Reaktionsaufarbeitung wird von gebildeten anorganischen Salzen abfiltriert. Das Filtrat wird eingeengt und gegebenenfalls chromatographisch oder durch Umkristallisation gereinigt.

Zur Durchführung des Verfahrens (D-β) setzt man pro Mol an Picolinamidin-Derivat der Formel (V) im allgemeinen 1 bis 3 Mol, vorzugsweise 1 bis 1,5 Mol an Acetal der Formel (VII) sowie im allgemeinen 0,01 bis 3, vorzugsweise 0,1 bis 1 Mol an Base ein.

Das Verfahren (D-γ) wird in Gegenwart von Basen durchgeführt. Vorzugsweise verwendet man Alkalimetallalkoholate wie z.B. Natriummethanolat und Natriumethanolat sowie organsicher Basen wie Triethylamin und N,N-Diethylanilin.

Zur Durchführung des Verfahrens (D-γ) setzt man pro Mol an Picolinamidin-Derivat der Formel (V) im allgemeinen, 1 bis 3 Mol, vorzugsweise 1 bis 1,5 Mol an 1,3-Dioxo-Derivat der Formel (VIII) sowie im allgemeinen 0,1 bis 5 Mol, vorzugsweise 0,1 bis 2,5 Mol an Base ein,

Zur Durchführung des Verfahrens (D-γ) wird die Reaktionsmischung mehrere Stunden bei der erforderlichen Temperatur gerührt.

Die Aufarbeitung und Isolierung des Reaktionsproduktes erfolgt nach üblichen Methoden.

Das Verfahren (D), Variante (δ) wird vorzugsweise in Abwesenheit von Verdünnungsmitteln durchgeführt. Es ist jedoch auch möglich eines der folgenden Verdünnungsmittel zu verwenden: aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem Verfahren (D) Variante (δ) in einem größeren Bereich variiert werden. Im allgemeinen arbeitet man bei Temperaturen zwischen 0 °C und +200 °C, vorzugsweise bei Temperaturen zwischen +50 °C und +150 °C.

Zur Durchführung des Verfahrens (D-δ) setzt man pro Mol Picolinamidin-Derivat der Formel (V) im allgemeinen 1 bis 5 Mol, vorzugsweise 1 bis 3 Mol an Diacetal der Formel (IX) ein.

Zur Durchführung des Verfahrens (D-δ) wird die Reaktionsmischung mehrere Stunden bei der erforderlichen Temperatur gerührt.

Die Aufarbeitung erfolgt nach üblichen Methoden durch Einengen unter vermindertem Druck und anschließender chromatographischer Reinigung.

Erfindungsgemäß zu schützende technische Materialien sind nicht-lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können Im Rahmen der zu schützenden Materialien seien auch Teile von Produktionsanlagen, beispielsweise Kühlwasserkreisläufe, genannt, deren Funktion durch Vermehrung von Mikroorganismen beeinträchtigt werden kann. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäß zu verwendenden Verbindungen gegen Pilze, insbesondere Schimmelpilze, Holz dauerhaft verfärbende und holzzerstörende Pilze, sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,

Staphylococcus, wie Staphylococcus aureus,
ferner Süsswasser- und Meeresalgen.

Je nach Anwendungsgebiet können die erfindungsgemäß zu verwendenden Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Die Anwendungskonzentrationen der erfindungsgemäß zu verwendenden Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäß zu verwendenden Wirkstoffe können auch in Mischung mit anderen bekannten Wirkstoffen angewendet werden. Beispielsweise seien die folgenden Wirkstoffe genannt: Benzylalkoholmono(poly)hemiformal und andere Formaldehyd abspaltende Verbindungen, Benzimidazolylmethylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate, wie 2-Phenylphenol, (2,2'-Dihydroxy-5,5'-dichlor)-diphenylmethan und 3-Methyl-4-chlor-phenol, Organo-Zinnverbindungen, N-Trihalogenmethylthio-Verbindungen, wie Folpet, Fluorfolpet und Dichlofluanid, Azolfungizide wie Tebuconazol, Propiconazol und Azaconazol, Iodpropargylderivate, Isothiazolinonderivate.

Als andere Wirkstoffe kommen auch in Betracht Algizide, Molluskizide, Wirkstoffe gegen "sea animals", die sich auf Schiffsbodenanstrichen ansiedeln, Insektizide.

Prozentangaben in den nachfolgenden Beispielen bedeuten Gewichtsprozente.

## Anwendungsbeispiele

### Beispiel 1

Zum Nachweis der Wirksamkeit gegen Pilze werden die minimalen Hemm-Konzentrationen (MHK) von erfindungsgemäß zu verwendenden Wirkstoffen bestimmt:

Ein Agar, der aus Bierwürze und Pepton hergestellt wird, wird mit Wirkstoff in Konzentrationen von 0,1 mg/l bis 5 000 mg/l versetzt. Nach Erstarren des Agars erfolgt Kontamination mit Reinkulturen der in der Tabelle aufgeführten Testorganismen. Nach 2-wöchiger Lagerung bei 28 °C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle angegeben.

Minimale Hemmkonzentrationen (MHK) in mg/l

| Testorganismen | Testsubstanz-Nr. 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Scopulariopsis brevicaule | 400 | 400 | 100 | 100 | 400 | 400 | 400 | 400 | 100 | 250 | 100 | 400 | 100 | 100 |
| Chaetomium globosum | 50 | 50 | 50 | 50 | 50 | 15 | 400 | 20 | 50 | 50 | 75 | 100 | 50 | 50 |
| Aspergillus niger | 100 | 400 | 100 | 400 | 400 | 600 | 400 | 400 | 100 | 100 | 600 | 400 | 600 | 100 |
| Lentinus tigrinus | 50 | 50 | 50 | 75 | 100 | 15 | 50 | 50 | 50 | 50 | 50 | 75 | 400 | 50 |
| Sclerophoma pityophila | 20 | 50 | 75 | 100 | 100 | 75 | 20 | 75 | 250 | 100 | 75 | 50 | 50 | 100 |
| Trichoderma viride | 250 | 400 | 400 | 50 | 400 | 600 | 400 | 400 | 400 | 400 | 400 | 250 | 250 | 100 |
| Cladosporium herbarum | 100 | 100 | 75 | 100 | 100 | 800 | 50 | 50 | 400 | 250 | 250 | 100 | 50 | 50 |
| Alternaria alternata | 400 | 400 | 400 | 400 | 400 | 35 | 400 | 400 | 400 | 400 | 400 | 100 | 250 | 100 |
| Aureobasidium pullulans | 100 | 400 | 250 | 400 | 250 | 75 | 250 | 250 | 400 | 400 | 250 | 100 | 75 | 100 |

Beispiel 2

Wirkung gegen Bakterien

Ein Agar, der als Nährmedium Bouillon enthält, wird mit Wirkstoff in Konzentrationen von 1 bis 5 000 ppm versetzt. Darauf infiziert man das Nährmedium jeweils mit den in Tabelle II aufgeführten Testorganismen und hält das infizierte Medium 2 Wochen bei 28° C und 60 bis 70 % rel. Luftfeuchtigkeit. Die MHK ist

die niedrigste Konzentration an Wirkstoff, bei der keinerlei Bewuchs durch die verwendete Mikrobenart erfolgt. Die MHK-Werte sind in Tabelle II wiedergegeben.

Tabelle II

Angabe der MHK-Werte in mg/l bei der Einwirkung der unten angegebenen Wirkstoffe auf Bakterien.

| Testorganismen | MHK in mg/l der Wirkstoffe | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| Staphylococcus aureus | 100 | 50 | 50 | 50 | 50 | 100 | 50 | 100 | 20 | 20 | 50 | 5 |

Beispiel 3

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaedodactylum tricornutum Bohlin) wird unter Durchperlen von Luft in Allens Nährlösung [Arch. Mikrobiol. 17, 34 bis 53 (1952)], die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt, wenn keine das Algenwachstum hemmende Wirkstoffkonzentration vorliegt.

Minimale Hemmkonzentration (MHK) von Testsubstanzen für Algen

| | |
|---|---|
| Testsubstand-Nr.: | 9 |
| MHK in mg/l: | 1 |

Beispiel 4 (Fungizide Wirkung in Anstrichmitteln)

Die fungizide Wirkung in Ansrichmitteln wird durch Prüfung der Schimmelfestigkeit der mit den Anstrichmitteln erhaltenen Anstriche bestimmt.

Die Prüfung wird in Anlehnung an den Report 219 der Defense Standards Laboratories Maribyrnong/Australien wie folgt durchgeführt:

Das zu prüfende Anstrichmittel wird beidseitig auf eine geeignete Unterlage gestrichen.

Um praxisnahe Ergebnisse zu erhalten, wird ein Teil der Prüflinge vor dem Test mit einem warmen Frischluftstrom behandelt (7 Tage; 40 °C).

Die so vorbereiteten Prüflinge werden auf einen Agar-Nährboden gelegt. Prüfling und Nährboden werden mit Pilzsporen kontaminiert. Nach 1- bis 3-wöchiger Lagerung bei 29 ± 1 °C und 80 bis 90 % rel. Luftfeuchte wird abgemustert. Der Anstrich ist dauerhaft schimmelfest, wenn der Prüfling pilzfrei bleibt oder höchstens einen geringen Randbefall erkennen läßt.

Zur Kontamination werden Pilzsporen folgender neun Schimmelpilze verwendet, die als Anstrichzerstörer bekannt sind oder häufig auf Anstrichen angetroffen werden:

1. Alternaria tenuis
2. Aspergillus flavus

3. Aspergillus niger
4. Aspergillus ustus
5. Cladosporium herbarum
6. Paecilomyces variotii
7. Penicillium citrinum
8. Aureobasidium pullulans
9. Stachybotrys atra Corda

In Proben einer handelsüblichen Dispersionsfarbe auf Polyvinylacetatbasis wurden 0 bis 2 Gew.-%, bezogen auf den Gesamtfeststoffgehalt der Farbe, der auf ihre fungizide Wirksamkeit zu prüfenden Verbindung eingearbeitet. Bei diesem Test lieferten bereits die Proben, die nur 0,6 Gew.-%, bezogen auf Gesamtfeststoffgehalt der Farbe, des Pyridinylpyrimidinderivates Nr. 9 enthielten, dauerhaft schimmelfeste Anstriche.

**Patentansprüche**

**1.** Verwendung von Verbindungen der Formel

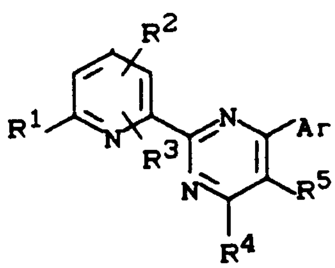

(I)

in welcher

$R^1$ für Wasserstoff, Halogen, Alkyl, Alkoxy, Alkylthio, Halogenalkyl, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist;

in welcher weiterhin

$R^1$ für jeweils gegebenenfalls substituiertes Aryloxy, Arylthio, Aralkyloxy oder Aralkylthio steht,

$R^2$ und $R^3$ unabhängig voneinander, gleich oder verschieden jeweils für Wasserstoff, Halogen, Alkyl, Halogenalkyl, Alkoxy, Alkylthio oder Alkoxycarbonyl stehen,

oder

$R^2$ und $R^3$ gemeinsam für eine Alkylenkette mit 3 bis 6 Kohlenstoffatomen stehen, die über die Ringpositionen 3 und 4 oder 4 und 5 verbunden sind,

$R^4$ für Wasserstoff, Halogen, Alkyl, Halogenalkyl, Hydroxy, Alkoxy, Mercapto, Alkylthio, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist; oder

$R^4$ für Alkenyloxy oder Alkinyloxy steht,

$R^5$ für Wasserstoff oder Alkyl steht und

Ar für unsubstituiertes oder substituiertes Aryl oder unsubstituiertes oder substituiertes Hetaryl steht,

als Mikrobizide zum Schutz technischer Materialien.

**2.** Verwendung nach Anspruch 1, dadurch gekennzeichnet, daß die technischen Materialien Anstrichmittel, Kunststoffe oder Kunststoffartikel sind.

Europäisches Patentamt

# EUROPÄISCHER RECHERCHENBERICHT

Nummer der Anmeldung

EP 92 10 8333

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| --- | --- | --- | --- |
| X | EP-A-0 407 899 (HOECHST AKTIENGESELLSCHAFT) * Seite 1, Zeile 6 - Seite 2, Zeile 48 * * Seite 4, Zeile 27 - Seite 4, Zeile 38; Ansprüche; Tabelle A * --- | 1,2 | A01N43/54 |
| P,A | EP-A-0 431 424 (BAYER AG) --- | | |
| P,A | EP-A-0 431 421 (BAYER AG) --- | | |
| A | EP-A-0 270 362 (SUMITOMO CHEMICAL COMPANY LIMITED) ----- | | |

RECHERCHIERTE SACHGEBIETE (Int. Cl.5 )

C07D
A01N
C07D

Der vorliegende Recherchenbericht wurde für alle Patentansprüche erstellt

| Recherchenort | Abschlußdatum der Recherche | Prüfer |
| --- | --- | --- |
| DEN HAAG | 02 SEPTEMBER 1992 | DONOVAN T.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)